(19) Europäisches Patentamt
European Patent Office
Office européen des brevets

(11) Veröffentlichungsnummer: **0 177 913**
**B1**

(12) # EUROPÄISCHE PATENTSCHRIFT

(45) Veröffentlichungstag der Patentschrift: **03.01.90**

(21) Anmeldenummer: **85112646.6**

(22) Anmeldetag: **05.10.85**

(51) Int. Cl.$^5$: **C 07 D409/12**, C 07 D407/12, C 07 D413/12, C 07 D333/28, C 07 D333/22, C 07 D493/04, C 07 D261/08, A 01 N 43/18, A 01 N 43/16, A 01 N 43/80, A 01 N 43/10 // (C07D493/04, 311:00, 311:00)

(54) **Cyclohexenonderivate, Verfahren zu ihrer Herstellung und ihre Verwendung zur Bekämpfung unerwünschten Pflanzenwuchses.**

(30) Priorität: **11.10.84 DE 3437238**

(43) Veröffentlichungstag der Anmeldung: **16.04.86 Patentblatt 86/16**

(45) Bekanntmachung des Hinweises auf die Patenterteilung: **03.01.90 Patentblatt 90/01**

(84) Benannte Vertragsstaaten: **BE CH DE FR GB IT LI NL**

(56) Entgegenhaltungen:
**EP-A- 0 066 195**
**EP-A- 0 070 370**
**EP-A- 0 071 707**
**EP-A- 0 107 156**
**US-A- 4 432 786**
**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Keil, Michael, Dr.**
**Fontanestrasse 4**
**D-6713 Freinsheim (DE)**
Erfinder: **Becker, Rainer, Dr.**
**Im Haseneck 22**
**D-6702 Bad Duerkheim (DE)**
Erfinder: **Jahn, Dieter, Dr.**
**Burgunder Weg 8**
**D-6803 Edingen-Neckarhausen (DE)**
Erfinder: **Kolassa, Dieter, Dr.**
**Moltkestrasse 8**
**D-6700 Ludwigshafen (DE)**
Erfinder: **Schirmer, Ulrich, Dr.**
**Berghalde 79**
**D-6900 Heidelberg (DE)**
Erfinder: **Will, Wolfgang, Dr.**
**Schulstrasse 46**
**D-6800 Mannheim 24 (DE)**
Erfinder: **Wuerzer, Bruno, Dr.**
**Ruedigerstrasse 13**
**D-6701 Otterstadt (DE)**
Erfinder: **Meyer, Norbert, Dr.**
**Dossenheimer Weg 22**
**D-6802 Ladenburg (DE)**

**EP 0 177 913 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Cyclohexenonderivate, Verfahren zu ihrer Herstellung, Herbizide, die diese Verbindungen enthalten, sowie ein Verfahren zur Bekämpfung unerwünschten Pflanzenwuchses.

Aus der DE-A-3 121 355, DE-A-3 123 312 und DE-A-3 239 071 sind eine Reihe von Cyclohexenonderivaten bekannt, die herbizid wirksam sind.

Speziell sind aus der US-A 4 432 786 Cyclohexenonderivate bekannt, die in 5-Stellung des Cyclohexenonrings u. a. eine Phenylgruppe als Substituenten tragen. Diese Verbindungen weisen jedoch nur eine unzureichende herbizide Wirkung auf.

Es wurden nun neue Cyclohexenonderivate der Formel I

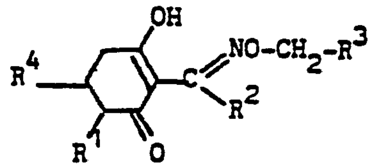

(I)

in der

$R^1$ Wasserstoff oder Alkoxycarbonyl mit 2 bis 5 Kohlenstoffatomen,

$R^2$ Alkyl mit 1 bis 4 Kohlenstoffatomen,

$R^3$ einen 5-gliedrigen Heterocyclus, der 1 bis 3 Heteroatome der Gruppe N, O, S, gegebenenfalls bis zu 2 Doppelbindungen und gegebenenfalls bis zu 2 Substituenten aus der Gruppe $C_1$-$C_4$-Alkyl, $C_1$-$C_4$-Alkoxy, Halogen, Trifluormethyl, $C_1$-$C_4$-Alkoxymethyl, $C_1$-$C_4$-Alkylthiomethyl, Vinyl oder Phenyl enthält,

$R^4$ einen 5- bis 7-gliedrigen Heterocyclus, der 1 oder 2 gleiche oder verschiedene Heteroatome oder Ringglieder aus der Gruppe N, O, S, SO und $SO_2$, und gegebenenfalls bis zu 3 Doppelbindungen enthält, und der gegebenenfalls durch maximal 2 Alkyl- oder Alkoxygruppen mit jeweils 1 bis 4 Kohlenstoffatomen substituiert ist oder einen Rest der Formel II

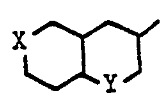

(II)

oder der Formel IIa

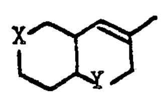

(IIa)

in denen X und Y jeweils für N, O, S, SO oder $SO_2$ stehen, bedeutet, und Salze dieser Verbindungen gefunden.

Die neuen Cyclohexenonderivate zeigen eine besonders gute, in vielen Fällen auch artspezifische herbizide Wirkung.

Die Verbindungen der Formel I können in mehreren tautomeren Formen auftreten, die alle vom Patentanspruch umfaßt werden :

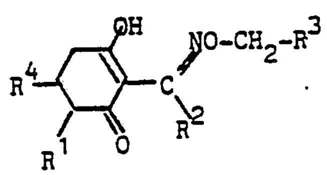

.

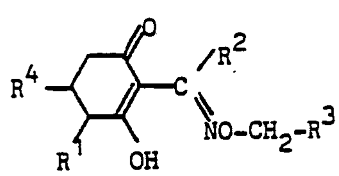

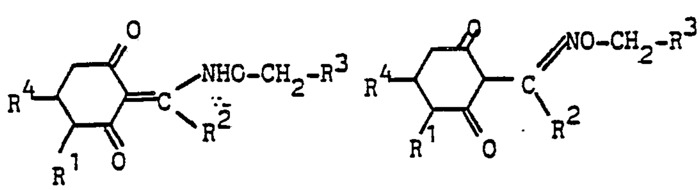

$R^1$ in Formel F bedeutet Wasserstoff oder Alkoxycarbonyl mit 1 bis 4 Kohlenstoffatomen, beispielsweise Methoxycarbonyl, Ethoxycarbonyl, Propoxycarbonyl oder Butoxycarbonyl. Bevorzugte Reste $R^1$

sind Methoxycarbonyl und insbesondere Wasserstoff.

$R^2$ in Formel I steht für Methyl, Ethyl, n-Propyl, i-Propyl, n-Butyl, sec-Butyl, i-Butyl oder tert-Butyl, wobei diejenigen Reste, die 2 und 3 Kohlenstoffatome aufweisen, bevorzugt sind.

Der Substituent $R^3$ ist ein 5-gliedriger heterocyclischer Ring mit 1 bis 3 Heteroatomen der Gruppe N, O, S, wobei Heterocyclen, die mehr als zwei Sauerstoffatome oder mehr als ein Schwefelatom im Ring aufweisen, ausgeschlossen sind. Die heterocyclischen Ringe können bis zu 2 Doppelbindungen und maximal 2 Substituenten wie $C_1$-$C_4$-Alkyl, $C_1$-$C_4$-Alkoxy, Halogen, Trifluormethyl, $C_1$-$C_4$-Alkoxymethyl, $C_1$-$C_4$-Alkylthiomethyl, Vinyl oder Phenyl aufweisen. Beispiele solcher Substituenten, die besonders günstige Wirkungen hervorrufen, sind Furan-2-yl, Tetrahydrofuran-2-yl, 1,3-Dioxolan-2-yl, 2-Phenyl-1,3-dioxolan-2-yl, 2,2-Dimethyl-1,3-dioxolan-4-yl, 4,5-Dimethyl-1,3-dioxolan-2-yl, 2-Vinyl-1,3-dioxolan-4-yl, 3-Phenyl-isoxazol-5-yl, 3-Methyl-isoxazol-5-yl, 3-Methoxymethyl-isoxazol-5-yl, 5-Chlorthien-2-yl, 2-Chlorthien-3-yl, 3,5-Dichlorthien-2-yl, 2,5-Dichlorthien-3-yl, 2,3-Dichlorthien-4-yl, 2-Chlorthien-4-yl, 2-Isopropyl-1,3,4-oxadiazol-5-yl, 2-Methyl-1,3,4-thiadiazol-5-yl, 5-Chlor-1,2,3-thiadiazol-4-yl, Thien-2-yl oder Thien-3-yl.

Besonders bevorzugte Reste $R^3$ sind gegebenenfalls jeweils durch 1 oder 2 Chloratome substituiertes Thien-2-yl und Thien-3-yl.

$R^4$ in Formel I bedeutet einen 5- bis 7-gliedrigen Heterocyclus, der 1 bis 2 Heteroatome oder Ringglieder aus der Gruppe N, O, S, SO und $SO_2$, und gegebenenfalls bis zu 3 Doppelbindungen enthält, und der gegebenenfalls durch maximal 2 Alkyl- oder Alkoxygruppen mit jeweils 1 bis 4 Kohlenstoffatomen substituiert ist, oder einen Rest der Formel II

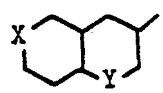

(II)

oder der Formel IIa

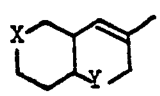

(IIa)

in denen X und Y, jeweils für N, O, S, SO oder $SO_2$ stehen.

Typische Beispiele für $R^4$ sind 4-Methyltetrahydropyran-3-yl, 3-Methyltetrahydropyran-4-yl, 2-Methyltetrahydropyran-4-yl, 2-Methoxytetrahydropyran-4-yl, 3-Methoxytetrahydropyran-4-yl, 2,6-Dimethyltetrahydrothiopyran-3-yl, 1-Oxo-tetrahydrothiopyran-3-yl, 1,1-Dioxotetrahydrothiopyran-3-yl, 2-Methyltetrahydrofuran-3-yl, 2,5-Dimethyltetrahydrofuran-3-yl, 2-Methoxymethyltetrahydrofuran-3-yl, Tetrahydrothien-3-yl, 2,2-Dimethyltetrahydrothien-3-yl, 2-Methyl-1,3-dioxepan-5-yl, 2-Isopropyl-1,3-dioxepan-5-yl, 4a,7,8,8a-Tetrahydro-2H,5H-thiino[4,3-b]pyran-3-yl und 3,4,4a,7,8,8a-Hexahydro-2H,5H-thiino-[4,3-b]pyran-3-yl. Bevorzugte Reste $R^4$ sind Tetrahydropyran-3-yl, Tetrahydropyran-4-yl, Tetrahydrothiopyran-3-yl, Tetrahydrothiopyran-4-yl, Tetrahydrofuran-3-yl, Tetrahydrothien-3-yl, Pyrid-3-yl, 1,3-Dioxepan-5-yl und die den Formeln II und IIa gehorchenden Reste 4a,7,8,8a-Tetrahydro-2H,5H-pyrano[4,3-b]pyran-3-yl, 3,4,4a,7,8,8a-Hexahydro-2H,5H-pyrano[4,3-b]pyran-3-yl, 4a,7,8,8a-Tetrahydro-2H,5H-thiino[4,3-b]-thiin-3-yl und 3,4,4a,7,8,8a-Hexahydro-2H,5H-thiino[4,3-b]thiin-3-yl.

Als Salze der Verbindung der Formel I kommen landwirtschaftlich brauchbare Salze, beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze sowie Ammonium-, Phosphonium-, Sylfoniumund Sulfoxoniumsalze, beispielsweise Ammoniumsalze, Tetraalkylammoniumsalze, Benzyltrialkylammoniumsalze, Trialkylsulfoniumsalze, Trialkylsulfoxoniumsalze in Betracht.

Die Cyclohexenonderivate der Formel I können durch Umsetzung von Tricarbonylverbindungen der Formel III

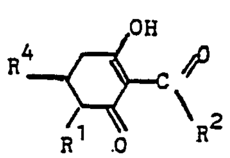

(III)

in der $R^1$, $R^2$ und $R^4$ die oben genannte Bedeutung besitzen, mit Ammoniumverbindungen der Formel $R^3$—$CH_2O$—$NH_3Y$, in der Y ein Anion (z. B. Chlorid, Bromid oder Sulfat) bedeutet, erhalten werden. Hierzu setzt man die beiden Reaktionsteilnehmer in einem Lösungsmittel in Gegenwart einer Base bei einer ausreichenden Temperatur, z. B. zwischen 0 und 80 °C, gegebenenfalls in Gegenwart von Wasser um. Geeignete Basen sind beispielsweise Carbonate, Hydrogencarbonate, Acetate, Alkoholate, Oxide bzw. Hydroxide von Alkali- oder Erdalkalimetallen, insbesondere von Natrium, Kalium, Magnesium, Calcium oder organische Basen, wie Pyridin oder tertiäre Amine wie Triethylamin.

Als Lösungsmittel sind beispielsweise Dimethylsulfoxid, Alkohole, wie Methanol, Ethanol, Isopropanol, Benzol, Toluol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Verteilung des Rückstandes in Methylenchlorid/Wasser und Abdestillieren des Lösungsmittels unter vermindertem Druck isoliert werden.

Die Verbindungen der Formel I können außerdem durch Umsetzen der Tricarbonylverbindungen der Formel III mit entsprechenden Alkoxylaminen der Formel $R^3—CH_2O—NH_2$, in einem geeigneten Lösungsmittel erhalten werden ; eine geeignete Reaktionstemperatur liegt im allgemeinen zwischen 15 und 70 °C. Das Alkoxylamin kann dabei auch in Form einer wäßrigen Lösung eingesetzt werden ; je nach dem verwendeten Lösungsmittel für den anderen Reaktionsteilnehmer erhält man ein- oder zweiphasiges Reaktionsgemisch.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Alkalimetallsalze der Verbindungen der Formel I können durch Behandeln dieser Verbindungen mit Natrium- oder Kaliumhydroxid oder -alkoholat in wäßriger Lösung oder in einem organischen Lösungsmittel, wie Methanol, Ethanol, Aceton, Toluol, erhalten werden.

Andere Metallsalze, z. B. die Mangan-, Kupfer-, Zink-, Eisen-, Calcium-, Magnesium- und bariumsalze können aus den Natriumsalzen in üblicher Weise hergestellt werden, ebenso Ammonium- und Phosphoniumsalze mittels Ammoniak, Phosphonium-, Sulfonium- oder Sulfoxoniumhydroxiden.

Die Tricarbonylverbindungen der Formel III sind im allgemeinen bekannt. Sie können z. B. aus entsprechenden Cyclohexan-1,3-dionen (IV) bzw. (IVa) bzw. (IVb)

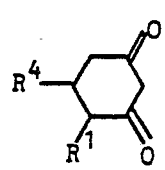
(III)

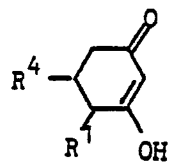
(IVa)

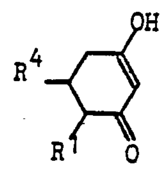
(IVb)

in bekannter Weise (Tetrahydron Letters 29, 2491 (1975)) hergestellt werden.

Sie lassen sich also auch über die Zwischenstufe der Enolester, die bei der Acylierung von Verbindungen (IV) eventuell als Isomerengemische anfallen und in Gegenwart von Imidazol- oder Pyridinderivaten umgelagert werden (JP-A-63 052/1979), herstellen.

Zu den Verbindungen der Formel (IV) gelangt man ebenfalls auf bekannte Weise wie dies aus folgendem Schema hervorgeht :

(Siehe Formel Seite 5 f.)

Das folgende Beispiel erläutert die Herstellung der Cyclohexenonderivate der Formel I :

## Beispiel

2,82 g (10 mmol) 2-Butyryl-3-hydroxy-5-(tetrahydrothiopyran-3-yl)-cylohex-2-en-1-on wurden zusammen mit 1,69 g (11 mmol) Tetrahydrofur-2-yl-methoxyaminhydrochlorid und 0,93 g (11 mmol) Natriumhydrogencarbonat in 20 ml Methanol 12 Stunden bei 25 °C gerührt. Die Mischung wurde eingeengt, mit Dichlormethan aufgenommen und mit Wasser ausgeschüttelt. Nach Einengen der getrockneten Dichlormethan-Lösung wurden 3,1 g (8,1 mmol) 3-Hydroxy-2-(1-tetrahydrofur-2-ylmethoxyiminobutyl)-5-(tetrahydrothiopyran-3-yl)-cyclohex-2-en-1-on als viskoses Öl erhalten

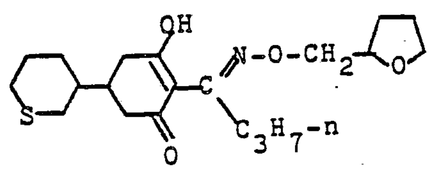

Ber. C 62,96 H 8,19 N 3,67
Gef. C 63,0 H 7,9 N 4,1

Weitere erfindungsgemäße Cyclohexenone sind in der nachstehenden Tabelle aufgeführt ; dabei

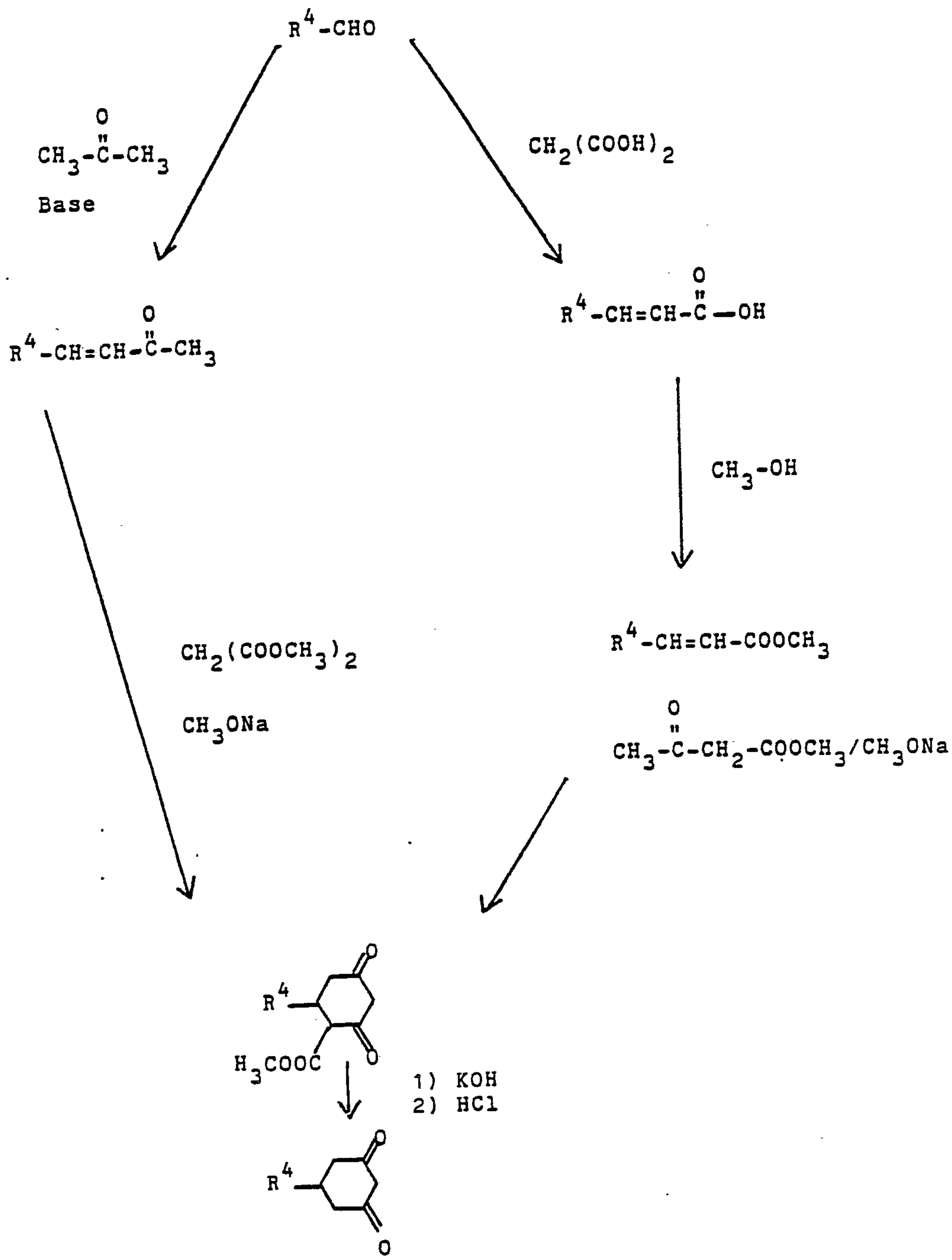
CH3-C(O)-CH3
Base
CH2(COOH)2
CH3-OH
CH2(COOCH3)2
CH3ONa
CH3-C(O)-CH2-COOCH3/CH3ONa
1) KOH
2) HCl

bedeutet

A : 2-Furyl
B : Tetrahydrofuran-2-yl
C : 1,3-Dioxolan-2-yl
D : 2-Phenyl-1,3-dioxolan-2-yl
E : 2,2-Dimethyl-1,3-dioxolan-4-yl
F : 4,5-Dimethyl-1,3-dioxolan-2-yl
G : 2-Vinyl-1,3-dioxolan-4-yl
H : 3-Phenyl-isoxazol-5-yl
I : 3-Methyl-isoxazol-5-yl
K : 3-Methoxymethyl-isoxazol-5-yl
L : 5-Chlorthien-2-yl
M : 2-Chlorthien-3-yl
N : 3,5-Dichlorthien-2-yl
O : 2,5-Dichlorthien-3-yl
P : 2,3-Dichlorthien-4-yl
Q : 2-Chlorthien-4-yl
R : 2-Isopropyl-1,3,4-oxadiazol-5-yl
S : 2-Methyl-1,3,4-thiadiazol-5-yl
T : 5-Chlor-1,2,3-thiadiazol-4-yl
U : Thien-2-yl
V : Thien-3-yl
W : 5-Bromthien-2-yl
X : 4-Bromthien-2-yl
Y : 5-Bromthien-3-yl

$^1$H-NMR-Spektren wurden in $CDCl_3$ mit TMS als internen Standard gemessen. In den Tabellen bedeutet s = Singulett, d = Dublett, t = Triplett, q = Quartett und m = Multiplett.

(Siehe Tabellen Seite 7 ff.)

Die Cyclohexenonderivate der Formel I sind bemerkenswert gute herbizide Wirkstoffe vorzugsweise gegen Arten aus der Familie der Gräser (Gramineen). Sie sind verträglich und somit selektiv in breitblättrigen Kulturen.

Die Anwendung als Herbizid erfolgt z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Streichen, Tränken, Vernebeln, Verstäuben, Verstreuen oder Gießen. Die Anwendungsformen richten sich ganz nach den Verwendungszwecken ; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, Methanol, Ethanol, Propanol, Butanol, Tetrachlorkohlenstoff, Cyclohexanol, Cyclohexanon, Chlorbenzol, Isophoran, stark polare Lösungsmittel, z. B. N,N-Dimethylformamid, Dimethylsulfoxid, N-Methylpyrrolidon oder Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen : Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, oder Nonylphenol, Alkylphenolpolyglykolether, Tributylphenylpolyglykolether, Alkylarylpolyetheralkoholate, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

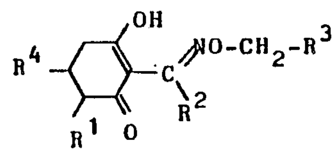

| Nr. | $R^4$ | $R^1$ | $R^2$ | $R^3$ | Charakteristische Signale $^1H$-NMR (ppm) |
|---|---|---|---|---|---|
| 1 | Tetrahydropyran-3-yl | $COOCH_3$ | $n-C_3H_7$ | L | |
| 2 | Tetrahydropyran-3-yl | H | $n-C_3H_7$ | B | 0,96(3H,t); 1,2-2,8(16H); 2,88(2H,t); 3,25(2H,m); 3,7-4,3(7H) |
| 3 | Tetrahydropyran-3-yl | H | $C_2H_5$ | L | |
| 4 | Tetrahydropyran-3-yl | H | $n-C_3H_7$ | U | 3,9 (t); 5,2 (s); 7,35 (m) |
| 5 | Tetrahydropyran-3-yl | H | $n-C_3H_7$ | L | 0,9 (t); 3,9 (t); 5,1 (s) |
| 6 | Tetrahydropyran-3-yl | H | $n-C_3H_7$ | P | 0,95 (t); 5,0 (s); 7,2 (s) |
| 7 | Tetrahydropyran-4-yl | $COOCH_3$ | $n-C_3H_7$ | L | |
| 8 | Tetrahydropyran-4-yl | $COOCH_3$ | $n-C_3H_7$ | M | |
| 9 | Tetrahydropyran-4-yl | H | $n-C_3H_7$ | V | 2,25 (t); 5,1 (s); 7,1 (m) |
| 10 | Tetrahydropyran-4-yl | H | $n-C_3H_7$ | L | 2,9 (q); 5,1 (s); 6,8 (m) |
| 11 | Tetrahydropyran-4-yl | H | $n-C_3H_7$ | M | |
| 12 | Tetrahydropyran-4-yl | H | $n-C_3H_7$ | N | |
| 13 | Tetrahydropyran-4-yl | H | $n-C_3H_7$ | O | |
| 14 | Tetrahydropyran-4-yl | H | $n-C_3H_7$ | P | 3,35 (t); 4,0 (d); 4,9 (s) |
| 15 | Tetrahydropyran-4-yl | H | $n-C_3H_7$ | Q | 3,35 (t); 4,0 (d); 6,9 (s) |
| 16 | Tetrahydropyran-4-yl | H | $C_2H_5$ | U | 3,4 (t); 5,2 (s); 7,3 (m) |
| 17 | Tetrahydropyran-4-yl | H | $C_2H_5$ | B | 1,1 (t); 1,6 (d); 3,4 (t) |
| 18 | Tetrahydropyran-4-yl | H | $C_2H_5$ | C | |
| 19 | Tetrahydropyran-4-yl | H | $C_2H_5$ | D | |

(Fortsetzung)

| Nr. | $R^4$ | $R^1$ | $R^2$ | $R^3$ | Charakteristische Signale $^1H$-NMR (ppm) |
|---|---|---|---|---|---|
| 20 | Tetrahydropyran-4-yl | H | $C_2H_5$ | E | |
| 21 | Tetrahydropyran-4-yl | H | $C_2H_5$ | F | |
| 22 | Tetrahydropyran-4-yl | H | $C_2H_5$ | G | |
| 23 | Tetrahydropyran-4-yl | H | $C_2H_5$ | V | 2,9 (q); 5,1 (s); 7,1 (d) |
| 24 | Tetrahydropyran-4-yl | H | $C_2H_5$ | I | 1,1 (t); 2,3 (t); 5,1 (s); 6,1 (s) |
| 25 | Tetrahydropyran-4-yl | H | $C_2H_5$ | K | |
| 26 | Tetrahydropyran-4-yl | H | $C_2H_5$ | L | 1,65 (d); 3,3 (t); 5,1 (s) |
| 27 | Tetrahydropyran-4-yl | H | $C_2H_5$ | M | |
| 28 | Tetrahydropyran-4-yl | H | $C_2H_5$ | N | |
| 29 | Tetrahydropyran-4-yl | H | $C_2H_5$ | O | |
| 30 | Tetrahydropyran-4-yl | H | $C_2H_5$ | P | |
| 31 | Tetrahydropyran-4-yl | H | $C_2H_5$ | Q | 3,4 (t); 4,9 (s); 6,9 (s) |
| 32 | Tetrahydropyran-4-yl | H | $C_2H_5$ | R | |
| 33 | Tetrahydropyran-4-yl | H | $C_2H_5$ | S | |
| 34 | Tetrahydropyran-4-yl | H | $C_2H_5$ | T | |
| 35 | 4-Methyltetrahydropyran-3-yl | H | n-$C_3H_7$ | L | 0,95(t); 1,5(q); 2,85(t); 3,2(t); 3,4(t); 5,1(s) |
| 36 | 4-Methyltetrahydropyran-3-yl | H | n-$C_3H_7$ | I | 0,94(3H,t); 0,98(3H,d); 2,31(3H,s); 2,84(2H,t); 5,09(2H,s); 6,16(1H,s) |
| 37 | 4-Methyltetrahydropyran-3-yl | H | $C_2H_5$ | L | |
| 38 | 4-Methyltetrahydropyran-3-yl | H | $C_2H_5$ | M | |
| 39 | 3-Methyltetrahydropyran-4-yl | H | $C_2H_5$ | L | |
| 40 | 3-Methyltetrahydropyran-4-yl | H | $C_2H_5$ | M | |
| 41 | 3-Methyltetrahydropyran-4-yl | H | n-$C_3H_7$ | L | |
| 42 | 3-Methyltetrahydropyran-4-yl | H | n-$C_3H_7$ | M | |
| 43 | 2-Methyltetrahydropyran-4-yl | H | n-$C_3H_7$ | L | |
| 44 | 2-Methyltetrahydropyran-4-yl | H | n-$C_3H_7$ | M | |

(Fortsetzung)

| Nr. | $R^4$ | $R^1$ | $R^2$ | $R^3$ | Charakteristische Signale $^1H$-NMR (ppm) |
|---|---|---|---|---|---|
| 45 | 2-Methyltetrahydropyran-4-yl | H | n-$C_3H_7$ | N | |
| 46 | 2-Methyltetrahydropyran-4-yl | H | n-$C_3H_7$ | E | |
| 47 | 2-Methyltetrahydropyran-4-yl | H | n-$C_3H_7$ | T | |
| 48 | 2-Methoxytetrahydropyran-4-yl | H | n-$C_3H_7$ | T | |
| 49 | 2-Methoxytetrahydropyran-4-yl | H | n-$C_3H_7$ | L | |
| 50 | 2-Methoxytetrahydropyran-4-yl | H | n-$C_3H_7$ | M | |
| 51 | 3-Methoxytetrahydropyran-4-yl | H | n-$C_3H_7$ | L | |
| 52 | 3-Methoxytetrahydropyran-4-yl | H | n-$C_3H_7$ | M | |
| 53 | Tetrahydrothiopyran-3-yl | H | n-$C_3H_7$ | U | 2,6 (m); 3,0 (t); 5,2 (s) |
| 54 | Tetrahydrothiopyran-3-yl | H | n-$C_3H_7$ | B | 0,96(3H,t); 1,2-2,7(20H); 2,88(2H,t); 3,84(2H,m); 4,09(3H) |
| 55 | Tetrahydrothiopyran-3-yl | H | n-$C_3H_7$ | C | |
| 56 | Tetrahydrothiopyran-3-yl | H | n-$C_3H_7$ | D | |
| 57 | Tetrahydrothiopyran-3-yl | H | n-$C_3H_7$ | E | |
| 58 | Tetrahydrothiopyran-3-yl | H | n-$C_3H_7$ | F | |
| 59 | Tetrahydrothiopyran-3-yl | H | n-$C_3H_7$ | G | |
| 60 | Tetrahydrothiopyran-3-yl | H | n-$C_3H_7$ | H | 0,88(t); 5,19(s); 6,72(s); 7,42(m); 7,8(m) |
| 61 | Tetrahydrothiopyran-3-yl | H | n-$C_3H_7$ | I | 0,94(3H,t); 2,31(3H,s); 2,84(2H,t); 3,00(1H,t); 5,09(2H,s); 6,16(1H,s) |
| 62 | Tetrahydrothiopyran-3-yl | H | n-$C_3H_7$ | V | 0,85 (t); 4,93 (s); 6,92 (s); 7,07 (s) |
| 63 | Tetrahydrothiopyran-3-yl | H | n-$C_3H_7$ | L | 0,96(3H,t); 2,84(2H,t); 3,01(1H,t); 5,06(2H,s); 6,79(2H) |
| 64 | Tetrahydrothiopyran-3-yl | H | n-$C_3H_7$ | M | |
| 65 | Tetrahydrothiopyran-3-yl | H | n-$C_3H_7$ | N | |
| 66 | Tetrahydrothiopyran-3-yl | H | n-$C_3H_7$ | O | 0,87 (t); 4,93 (s); 6,98 (s) |
| 67 | Tetrahydrothiopyran-3-yl | H | n-$C_3H_7$ | P | 0,92 (t); 4,98 (s); 7,23 (s) |
| 68 | Tetrahydrothiopyran-3-yl | H | n-$C_3H_7$ | Q | 0,85 (t); 4,93 (s); 6,92 (s); 7,06 (s) |
| 69 | Tetrahydrothiopyran-3-yl | H | n-$C_3H_7$ | R | |

(Fortsetzung)

| Nr. | $R^4$ | $R^1$ | $R^2$ | $R^3$ | Charakteristische Signale $^1H$-NMR (ppm) |
|---|---|---|---|---|---|
| 70 | Tetrahydrothiopyran-3-yl | H | $n-C_3H_7$ | S | |
| 71 | Tetrahydrothiopyran-3-yl | H | $n-C_3H_7$ | T | |
| 72 | Tetrahydrothiopyran-4-yl | H | $n-C_3H_7$ | L | |
| 73 | Tetrahydrothiopyran-4-yl | H | $n-C_3H_7$ | M | |
| 74 | Tetrahydrothiopyran-4-yl | H | $n-C_3H_7$ | N | |
| 75 | Tetrahydrothiopyran-4-yl | H | $n-C_3H_7$ | O | |
| 76 | 2,6-Dimethyltetrahydrothiopyran-3-yl | H | $n-C_3H_7$ | A | |
| 77 | 2,6-Dimethyltetrahydrothiopyran-3-yl | H | $n-C_3H_7$ | B | |
| 78 | 2,6-Dimethyltetrahydrothiopyran-3-yl | H | $n-C_3H_7$ | C | |
| 79 | 1-Oxo-tetrahydrothiopyran-3-yl | H | $n-C_3H_7$ | C | |
| 80 | 1-Oxo-tetrahydrothiopyran-3-yl | H | $n-C_3H_7$ | E | |
| 81 | 1-Oxo-tetrahydrothiopyran-3-yl | H | $n-C_3H_7$ | L | 0,96 (t); 3,5 (m); 5,07 (s); 6,83 (m) |
| 82 | 1-Oxo-tetrahydrothiopyran-3-yl | H | $n-C_3H_7$ | M | |
| 83 | 1,1-Dioxo-tetrahydrothiopyran-3-yl | H | $n-C_3H_7$ | C | |
| 84 | 1,1-Dioxo-tetrahydrothiopyran-3-yl | H | $n-C_3H_7$ | E | |
| 85 | 1,1-Dioxo-tetrahydrothiopyran-3-yl | H | $n-C_3H_7$ | L | 0,95(t); 3,07(m); 5,09(s); 6,80(d); 6,88(d) |
| 86 | 1,1-Dioxo-tetrahydrothiopyran-3-yl | H | $n-C_3H_7$ | M | |
| 87 | Tetrahydrofuran-3-yl | H | $n-C_3H_7$ | P | 0,94 (t); 3,43 (t); 4,98 (s); 7,18 (s) |
| 88 | Tetrahydrofuran-3-yl | H | $n-C_3H_7$ | Q | 0,96(t); 2,88(t); 3,42(t); 4,92(s); 6,9(s); 7,05(s) |
| 89 | Tetrahydrofuran-3-yl | H | $n-C_3H_7$ | L | 0,94 (t); 5,07 (s); 6,82 (d); 6,88 (d) |
| 90 | Tetrahydrofuran-3-yl | H | $n-C_3H_7$ | M | |
| 91 | 2-Methyltetrahydrofuran-3-yl | H | $n-C_3H_7$ | L | 0,95 (t); 1,28 (d); 5,08 (s); 6,81 (d); 6,87 (d) |
| 92 | 2-Methyltetrahydrofuran-3-yl | H | $n-C_3H_7$ | M | |
| 93 | 2,5-Dimethyltetrahydrofuran-3-yl | H | $n-C_3H_7$ | M | |
| 94 | 2,5-Dimethyltetrahydrofuran-3-yl | H | $n-C_3H_7$ | L | |

(Fortsetzung)

| Nr. | $R^4$ | $R^1$ | $R^2$ | $R^3$ | Charakteristische Signale $^1H$-NMR (ppm) |
|---|---|---|---|---|---|
| 95 | 2-Methoxymethyltetrahydrofuran-3-yl | H | n-$C_3H_7$ | L | |
| 96 | 2-Methoxymethyltetrahydrofuran-3-yl | H | n-$C_3H_7$ | M | |
| 97 | Tetrahydrothien-3-yl | H | n-$C_3H_7$ | P | 0,95 (t); 4,97 (s); 7,18 (s) |
| 98 | Tetrahydrothien-3-yl | H | n-$C_3H_7$ | Q | 0,95 (t); 4,94 (s); 6,88 (s); 7,03 (s) |
| 99 | Tetrahydrothien-3-yl | H | n-$C_3H_7$ | L | |
| 100 | Tetrahydrothien-3-yl | H | n-$C_3H_7$ | M | |
| 101 | Tetrahydrothien-3-yl | H | $C_2H_5$ | U | 1,10t); 5,22(s); 7,03(m); 7,10(d); 7,35(d) |
| 102 | Tetrahydrothien-3-yl | H | $C_2H_5$ | V | 1,11 (t); 5,05 (s); 7,09 (d); 7,34 (m) |
| 103 | Tetrahydrothien-3-yl | H | $C_2H_5$ | L | |
| 104 | Tetrahydrothien-3-yl | H | $C_2H_5$ | M | |
| 105 | Tetrahydrothien-3-yl | H | $C_2H_5$ | N | |
| 106 | 2,2-Dimethyltetrahydrothien-3-yl | H | n-$C_3H_7$ | E | |
| 107 | 2,2-Dimethyltetrahydrothien-3-yl | H | n-$C_3H_7$ | I | 0,94(t); 1,34(s); 1,50(s); 5,09(s); 6,15(s) |
| 108 | 2,2-Dimethyltetrahydrothien-3-yl | H | n-$C_3H_7$ | L | |
| 109 | 2,2-Dimethyltetrahydrothien-3-yl | H | n-$C_3H_7$ | M | |
| 110 | 2,2-Dimethyltetrahydrothien-3-yl | H | $C_2H_5$ | E | |
| 111 | 2,2-Dimethyltetrahydrothien-3-yl | H | $C_2H_5$ | I | 1,12(t); 1,33(s); 1,49(s); 5,10(s); 6,15(s) |
| 112 | 2,2-Dimethyltetrahydrothien-3-yl | H | $C_2H_5$ | L | |
| 113 | 2,2-Dimethyltetrahydrothien-3-yl | H | $C_2H_5$ | M | |
| 114 | 3-Pyridyl | H | $C_2H_5$ | P | 1,14(t); 2,73(m); 2,9(q); 3,4(m);5,0(s) |
| 115 | 3-Pyridyl | H | $C_2H_5$ | I | 1,14(t); 2,3(s); 2,9(q); 3,4(m); 5,1(s); 6,18(s) |
| 116 | 3-Pyridyl | H | $C_2H_5$ | L | 1,16 (t); 2,9 (q); 5,1 (s) |
| 117 | 3-Pyridyl | H | $C_2H_5$ | M | |
| 118 | 3-Pyridyl | H | n-$C_3H_7$ | Q | 0,95 (t); 1,55 (q); 2,9 (t); 4,95 (s) |
| 119 | 3-Pyridyl | H | n-$C_3H_7$ | I | 1,00(3H,t); 2,31(3H,s); 3,63(1H,m); 6,02(1H,s); 7,25(1H,m); 7,58(1H,m); 8,50(2H,m) |

(Fortsetzung)

| Nr. | $R^4$ | $R^1$ | $R^2$ | $R^3$ | Charakteristische Signale $^1$H-NMR (ppm) |
|---|---|---|---|---|---|
| 120 | 3-Pyridyl | H | $n-C_3H_7$ | L | 0,95 (t); 1,55 (q); 5,1 (s) |
| 121 | 1,3-Dioxepan-5-yl | H | $n-C_3H_7$ | L | |
| 122 | 2-Methyl-1,3-dioxepan-5-yl | H | $n-C_3H_7$ | L | |
| 123 | 2-Isopropyl-1,3-dioxepan-5-yl | H | $C_2H_5$ | U | |
| 124 | 2-Isopropyl-1,3-dioxepan-5-yl | H | $C_2H_5$ | B | |
| 125 | 2-Isopropyl-1,3-dioxepan-5-yl | H | $C_2H_5$ | C | |
| 126 | 2-Isopropyl-1,3-dioxepan-5-yl | H | $C_2H_5$ | D | |
| 127 | 2-Isopropyl-1,3-dioxepan-5-yl | H | $C_2H_5$ | E | |
| 128 | 2-Isopropyl-1,3-dioxepan-5-yl | H | $C_2H_5$ | F | |
| 129 | 2-Isopropyl-1,3-dioxepan-5-yl | H | $C_2H_5$ | G | |
| 130 | 2-Isopropyl-1,3-dioxepan-5-yl | H | $C_2H_5$ | V | |
| 131 | 2-Isopropyl-1,3-dioxepan-5-yl | H | $C_2H_5$ | I | 0,9 (m); 2,3 (s); 5,1 (s) |
| 132 | 2-Isopropyl-1,3-dioxepan-5-yl | H | $C_2H_5$ | K | |
| 133 | 2-Isopropyl-1,3-dioxepan-5-yl | H | $C_2H_5$ | L | 0,9 (m); 2,9 (m); 5,1 (s) |
| 134 | 2-Isopropyl-1,3-dioxepan-5-yl | H | $C_2H_5$ | M | |
| 135 | 2-Isopropyl-1,3-dioxepan-5-yl | H | $C_2H_5$ | N | |
| 136 | 2-Isopropyl-1,3-dioxepan-5-yl | H | $C_2H_5$ | O | |
| 137 | 2-Isopropyl-1,3-dioxepan-5-yl | H | $C_2H_5$ | P | |
| 138 | 2-Isopropyl-1,3-dioxepan-5-yl | H | $C_2H_5$ | Q | 1,1 (t); 2,9 (q); 4,9 (s) |
| 139 | 2-Isopropyl-1,3-dioxepan-5-yl | H | $C_2H_5$ | R | |
| 140 | 2-Isopropyl-1,3-dioxepan-5-yl | H | $C_2H_5$ | S | |
| 141 | 2-Isopropyl-1,3-dioxepan-5-yl | H | $C_2H_5$ | T | |
| 142 | 4a,7,8,8a-Tetrahydro-2H,5H-pyrano-[4,3-b]pyran-3-yl | H | $n-C_3H_7$ | A | |

(Fortsetzung)

| Nr. | $R^4$ | $R^1$ | $R^2$ | $R^3$ | Charakteristische Signale $^1H$-NMR (ppm) |
|---|---|---|---|---|---|
| 143 | 4a,7,8,8a-Tetrahydro-2H,5H-pyrano-[4,3-b]pyran-3-yl | H | n-$C_3H_7$ | B | 0,98 (t); 4,1 (m); 4,2 (s); 5,3 (s) |
| 144 | 4a,7,8,8a-Tetrahydro-2H,5H-pyrano-[4,3-b]pyran-3-yl | H | n-$C_3H_7$ | C | |
| 145 | 4a,7,8,8a-Tetrahydro-2H,5H-pyrano-[4,3-b]pyran-3-yl | H | n-$C_3H_7$ | D | |
| 146 | 4a,7,8,8a-Tetrahydro-2H,5H-pyrano-[4,3-b]pyran-3-yl | H | n-$C_3H_7$ | E | |
| 147 | 4a,7,8,8a-Tetrahydro-2H,5H-pyrano-[4,3-b]pyran-3-yl | H | n-$C_3H_7$ | F | |
| 148 | 4a,7,8,8a-Tetrahydro-2H,5H-pyrano-[4,3-b]pyran-3-yl | H | n-$C_3H_7$ | G | |
| 149 | 4a,7,8,8a-Tetrahydro-2H,5H-pyrano-[4,3-b]pyran-3-yl | H | n-$C_3H_7$ | H | |
| 150 | 4a,7,8,8a-Tetrahydro-2H,5H-pyrano-[4,3-b]pyran-3-yl | H | n-$C_3H_7$ | I | 0,9(t); 2,3(s); 5,1(s); 5,35(s); 6,15(s) |
| 151 | 4a,7,8,8a-Tetrahydro-2H,5H-pyrano-[4,3-b]pyran-3-yl | H | n-$C_3H_7$ | U | 0,92(t); 5,2(s); 7,0(m); 7,1(d); 7,35(d) |
| 152 | 4a,7,8,8a-Tetrahydro-2H,5H-pyrano-[4,3-b]pyran-3-yl | H | n-$C_3H_7$ | L | 0,92(t); 4,2(s); 5,08(s); 5,3(s); 6,82(m) |
| 153 | 4a,7,8,8a-Tetrahydro-2H,5H-pyrano-[4,3-b]pyran-3-yl | H | n-$C_3H_7$ | M | |
| 154 | 4a,7,8,8a-Tetrahydro-2H,5H-pyrano-[4,3-b]pyran-3-yl | H | n-$C_3H_7$ | V | 0,95(t); 5,03(s); 5,3(s); 7,05(d); 7,3(m) |

(Fortsetzung)

| Nr. | $R^4$ | $R^1$ | $R^2$ | $R^3$ | Charakteristische Signale $^1H$-NMR (ppm) |
|---|---|---|---|---|---|
| 155 | 4a,7,8,8a-Tetrahydro-2H,5H-pyrano-[4,3-b]pyran-3-yl | H | n-$C_3H_7$ | O | |
| 156 | 4a,7,8,8a-Tetrahydro-2H,5H-pyrano-[4,3-b]pyran-3-yl | H | n-$C_3H_7$ | P | 0,93(t); 4,98(s); 5,35(s); 7,18(s) |
| 157 | 4a,7,8,8a-Tetrahydro-2H,5H-pyrano-[4,3-b]pyran-3-yl | H | n-$C_3H_7$ | Q | 0,93(t); 4,96(s); 5,35(s); 6,9(s); 7,05(s) |
| 158 | 4a,7,8,8a-Tetrahydro-2H,5H-pyrano-[4,3-b]pyran-3-yl | H | n-$C_3H_7$ | R | |
| 159 | 4a,7,8,8a-Tetrahydro-2H,5H-pyrano-[4,3-b]pyran-3-yl | H | n-$C_3H_7$ | S | |
| 160 | 4a,7,8,8a-Tetrahydro-2H,5H-pyrano-[4,3-b]pyran-3-yl | H | n-$C_3H_7$ | T | |
| 161 | 3,4,4a,7,8,8a-Hexahydro-2H,5H-pyrano-[4,3-b]pyran-3-yl | H | n-$C_3H_7$ | L | 0,92(t); 4,05(m); 5,08(s); 6,82(m) |
| 162 | 3,4,4a,7,8,8a-Hexahydro-2H,5H-pyrano-[4,3-b]pyran-3-yl | H | n-$C_3H_7$ | M | |
| 163 | 4a,7,8,8a-Tetrahydro-2H,5H-thiino-[4,3-b]pyran-3-yl | H | n-$C_3H_7$ | L | |
| 164 | 4a,7,8,8a-Tetrahydro-2H,5H-thiino-[4,3-b]pyran-3-yl | H | n-$C_3H_7$ | M | |
| 165 | 4a,7,8,8a-Tetrahydro-2H,5H-thiino-[4,3-b]pyran-3-yl | H | n-$C_3H_7$ | N | |
| 166 | 3,4,4a,7,8,8a-Hexahydro-2H,5H-thiino-[4,3-b]pyran-3-yl | H | n-$C_3H_7$ | L | |

(Fortsetzung)

| Nr. | $R^4$ | $R^1$ | $R^2$ | $R^3$ | Charakteristische Signale $^1H$-NMR (ppm) |
|---|---|---|---|---|---|
| 167 | 3,4,4a,7,8,8a-Hexahydro-2H,5H-thiino-[4,3-b]pyran-3-yl | H | n-$C_3H_7$ | M | |
| 168 | 4a,7,8,8a-Tetrahydro-2H,5H-thiino-[4,3-b]thiin-3-yl | H | n-$C_3H_7$ | L | |
| 169 | 4a,7,8,8a-Tetrahydro-2H,5H-thiino-[4,3-b]thiin-3-yl | H | n-$C_3H_7$ | M | |
| 170 | 4a,7,8,8a-Tetrahydro-2H,5H-thiino-[4,3-b]thiino-3-yl | H | n-$C_3H_7$ | N | |
| 171 | 4a,7,8,8a-Tetrahydro-2H,5H-thiino-[4,3-b]thiin-3-yl | H | n-$C_3H_7$ | O | |
| 172 | 4a,7,8,8a-Tetrahydro-2H,5H-thiino-[4,3-b]thiin-3-yl | H | $C_2H_5$ | L | |
| 173 | 4a,7,8,8a-Tetrahydro-2H,5H-thiino-[4,3-b]thiin-3-yl | H | $C_2H_5$ | M | |
| 174 | 4a,7,8,8a-Tetrahydro-2H,5H-thiino-[4,3-b]thiin-3-yl | H | $C_2H_5$ | N | |
| 175 | 3,4,4a,7,8,8a-Hexahydro-2H,5H-thiino-[4,3-b]thiin-3-yl | H | n-$C_3H_7$ | L | |
| 176 | 3,4,4a,7,8,8a-Hexahydro-2H,5H-thiino-[4,3-b]thiin-3-yl | H | n-$C_3H_7$ | M | |
| 177 | 3,4,4a,7,8,8a-Hexahydro-2H,5H-thiino-[4,3-b]thiin-3-yl | H | n-$C_3H_7$ | N | |
| 178 | 3,4,4a,7,8,8a-Hexahydro-2H,5H-thiino-[4,3-b]thiin-3-yl | H | $C_2H_5$ | L | |
| 179 | 3,4,4a,7,8,8a-Hexahydro-2H,5H-thiino-[4,3-b]thiin-3-yl | H | $C_2H_5$ | M | |
| 180 | 3,4,4a,7,8,8a-Hexahydro-2H,5H-thiino-[4,3-b]thiin-3-yl | H | $C_2H_5$ | N | |

(Fortsetzung)

| Nr. | $R^4$ | $R^1$ | $R^2$ | $R^3$ | Charakteristische Signale $^1$H-NMR (ppm) |
|---|---|---|---|---|---|
| 181 | 4a,7,8,8a-Tetrahydro-2H,5H-pyrano [4,3-b]pyran-3-yl | H | n-$C_3H_7$ | K | 0,92(t); 3,4(s); 4,55(s) 5,15(s); 5,35(s); 6,4(s) |
| 182 | Tetrahydropyran-3-yl | H | n-$C_3H_7$ | Q | 0,95(t); 4,9(s); 6,4(s); 7,05(s) |
| 183 | Tetrahydropyran-3-yl | H | $CH_3$ | U | 2,3(s); 3,2(t); 5,2(s) |
| 184 | Tetrahydrothiopyran-3-yl | H | n-$C_3H_7$ | W | 0,92(t); 5,09(s); 6,83(d); 6,94(d) |
| 185 | Tetrahydrothiopyran-3-yl | H | n-$C_3H_7$ | X | 0,83(t); 5,18(s); 7,11(s); 7,63(s) |
| 186 | Tetrahydrothiopyran-3-yl | H | n-$C_3H_7$ | Y | 0,95(t); 4,96(s); 7,03(s); 7,17(s) |
| 187 | Tetrahydropyran-3-yl | H | $CH_3$ | U | 0,95(t); 3,9(t); 6,9(s) |
| 188 | Tetrahydropyran-3-yl | H | n-$C_3H_7$ | Q | 2,3(s); 3,15(t); 5,2(s) |
| 189 | 3-Methoxymethylisoxazol-5-yl | H | n-$C_3H_7$ | V | 0,95(t); 3,4(s); 4,48(s); 4,95(s) |
| 190 | 3-Methoxymethylisoxazol-5-yl | H | n-$C_3H_7$ | Q | 0,95(t); 3,4(s); 4,48(s); 5,05(s); 6,1(s); |
| 191 | 3-Methoxymethylisoxazol-5-yl | H | n-$C_3H_7$ | I | 0,92(t); 2,34(s); 3,4(s); 4,5(s); 5,1(s); 6,3(s); 6,34(s) |

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, z. B. Ammoniumsulfat, Ammoniumphosphat, Ammoniumnitrat, Harnstoffe und pflanzliche Produkte, z. B. Getreidemehle, Baumrinden- Holz- und Nußschalenmehl, Cellulosepulver und andere feste Trägerstoffe.

Die Formulierungen enthalten zwischen 0,1 und 95 Gewichstprozent, vorzugsweise zwischen 0,5 und 90 Gewichtsprozent Wirkstoff.

Beispiele für Formulierungen sind :

I. Man vermischt 90 gewichtsteile der Verbindung 61 mit 10 Gewichtsteilen N-Methyl-alpha-pyrrolidon und erhält eine Lösung, die zur Anwendung in Form kleinster Tropfen geeignet ist.

II. 10 Gewichtsteile der Verbindung 61 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsaure-N-mono-ethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

III. 20 Gewichtsteile der Verbindung 36 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung 63 werden in einer Mischung gelöst, die aus 25 Gewichtsteilen Cyclohexanon, 65 gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht.

V. 80 Gewichtsteile des Wirkstoffs 63 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile der Verbindung 36 werden mit 95 Gewichtsteilen feinteiligem Kaolin innig vermischt. Man erhält auf diese Weise ein Stäubemittel, das 5 Gewichtsprozent des Wirkstoffs enthält.

VII. 30 Gewichtsprozent der Verbindung 63 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile des Wirkstoffs 61 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion.

IX. 20 Teile des Wirkstoffs 36 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Mineralöls innig vermischt. Man erhält eine stabile ölige Dispersion.

Die Applikation der Mittel, die diese Wirkstoffe enthalten, kann im Vorauflaufverfahren oder im Nachauflaufverfahren erfolgen. Wenn die Wirkstoffe für gewisse Kulturpflanzen weniger verträglich sind, können auch Ausbringungstechniken angewandt werden, bei welchen die herbiziden Mittel mit Hilfe der Spritzgeräte so gespritz werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,05 bis 5 kg/ha, vorzugsweise 0,1 bis 3 kg/ha.

Die Wirkung der Cyclohexenonderivate der Formel I auf das Pflanzenwachstum läßt sich durch Gewächshausversuche zeigen :

Als Kulturgefäße dienen Plastikblumentöpfe mit 300 $cm^3$ Inhalt und lehmigem Sand mit etwa 3,0 % Humus als Susbtrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 0,25, 0,5 und 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pfanzen angewaschen sind. Diese Abdeckung bewirkt ein gleichmäßiges Keimen der Testpflanzen, sofern dies nicht durch die Wirkstoffe beeinträchtigt wird.

Zum Zweck der Nachauflaufbehandlung zieht man die Testpflanzen je nach Wuchsform erst bis zu einer Wuchshöhe von 3 bis 15 cm an und behandelt sie danach. Zur Nachauflaufbehandlung werden

entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,25, 0,5 und 3 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

| Lat. Name | Deutscher Name |
|---|---|
| Avena sativa | Hafer |
| Echinochloa crus-galli | Hühnerhirse |
| Lolium multiflorum | Ital. Raygras |
| Mentha piperita | Pfefferminze |
| Sinapis alba | Weißer Senf |
| Medicago sativa | Luzerne |
| Hordeum vulgare | Gerste |
| Zea mays | Mais |
| Avena fatua | Flughafer |
| Setaria italica | Kolbenhirse |

Bei Vorauflaufanwendung erweisen sich die Beispielsverbindungen Nr. 61 und 36 als herbizid wirksam gegen Pflanzen aus der Familie der Gräser, während Sinapis alba als ein Vertreter dikotyler Pflanzen völlig ungeschädigt bleibt.

Im Nachauflaufverfahren werden mit den Beispielverbindungen Nr. 61 und 36 verschiedene Gräser sehr gut bekämpft ; an Mentha piperita treten keinerlei Schadsymptome auf.

Die Bekämpfung unerwünschter grasartiger Vegetation, die auch Ausfallkulturen einschließen kann, in Luzerne ist mit den Beispielverbindungen Nr. 61 und 63 möglich, ohne die Beispielskultur zu schädigen.

In Anbetracht des erfaßbaren Wirkungsspektrums zur Unkrautbekämpfung, der Verträglichkeit für Kulturpflanzen oder der unerwünschten Beeinflussung des Wachstums derselben sowie angesichts der Vielfalt der Applikations-methoden können die erfindungsgemäßen Verbindungen in einer großen Zahl von Kulturpflanzen eingesetzt werden.

In Betracht kommen beispielsweise folgende Kulturpflanzen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |

| | |
|---|---|
| Elaeis guineensis | Ölpalme |
| Fragaria vesca | Erdbeere |
| Glyoine max | Sojabohne |
| Gossypium hirsutum (Gossypium arboreum) | Baumwolle |
| Gossypium herbaceum (Gossypium vitifolium) | |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselium crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen Wirkstoffe sowohl unter sich als auch mit zahlreichen Vertretern anderer herbizider oder wachstumsregulierender Wirkstoffe gemischt und gemeinsam ausgebracht werden. Beispielsweise kommen als Mischungspartner Verbindungen aus den Gruppen der Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Cyclohexenonderivate der Formel I bzw. sie enthaltende

herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurelenelementmängeln eingesetzt werden. Es können auch nichtphytotoxische Öle und Ölkonzentrate zugesetzt werden.

**Patentansprüche**

1. Cyclohexenonderivate der Formel I

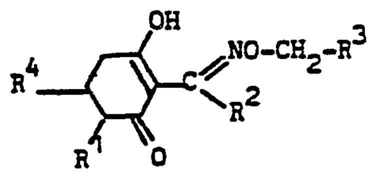

(I)

in der

$R^1$ Wasserstoff oder Alkoxycarbonyl mit 2 bis 5 Kohlenstoffatomen,

$R^2$ Alkyl mit 1 bis 4 Kohlenstoffatomen,

$R^3$ einen 5-gliedrigen Heterocyclus, der 1 bis 3 Heteroatome der Gruppe N, O, S, gegebenenfalls bis zu 2 Doppelbindungen und gegebenenfalls bis zu 2 Substituenten aus der Gruppe $C_1$-$C_4$-Alkyl, $C_1$-$C_4$-Alkoxy, Halogen, Trifluormethyl, $C_1$-$C_4$-Alkoxymethyl, $C_1$-$C_4$-Alkylthiomethyl, Vinyl oder Phenyl enthält,

$R^4$ einen 5- bis 7-gliedrigen Heterocyclus, der 1 oder 2 gleiche oder verschiedene Heteroatome oder Ringglieder aus der Gruppe N, O, S, SO und $SO_2$, und gegebenenfalls bis zu 3 Doppelbindungen enthält, und der gegebenenfalls durch maximal 2 Alkyl- oder Alkoxygruppen mit jeweils 1 bis 4 Kohlenstoffatomen substituiert ist, oder einen Rest der Formel II

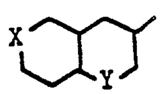

(II)

oder der Formel IIa

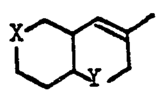

(IIa)

in denen X und Y jeweils für N, O, S, SO oder $SO_2$ stehen, bedeutet, und Salze dieser Verbindungen.

2. Cyclohexenonderivate der Formel I gemäß Anspruch 1, in denen $R^3$ einen Substituenten, ausgewählt aus der Gruppe, umfassend Furan-2-yl, Tetrahydrofuran-2-yl, 1,3-Dioxolan-2-yl, 2-Phenyl-1,3-dioxolan-2-yl, 2,2-Dimethyl-1,3-dioxolan-4-yl, 4,5-Dimethyl-1,3-dioxolan-2-yl, 2-Vinyl-1,3-dioxolan-4-yl, 3-Phenyl-isoxazol-5-yl, 3-Methyl-isoxazol-5-yl, 3-Methoxymethyl-isoxazol-5-yl, 5-Chlorothien-2-yl, 2-Chlorothien-3-yl, 3,5-Dichlorothien-2-yl, 2,5-Dichlorothien-3-yl, 2,3-Dichlorothien-4-yl, 2-Chlorothien-4-yl, 2-Isopropyl-1,3,4-oxadiazol-5-yl, 2-Methyl-1,3,4-thiadiazol-5-yl, 5-Chlor-1,2,3-thiadiazol-4-yl, Thien-2-yl und Thien-3-yl, bedeutet.

3. Cyclohexenonderivate der Formel I gemäß Anspruch 1, in denen $R^4$ einen Substituenten ausgewählt aus der Gruppe, umfassend Tetrahydropyran-3-yl, Tetrahydropyran-4-yl, Tetrahydrothiopyran-3-yl, Tetrahydrothiopyran-4-yl, Tetrahydrofuran-3-yl, Tetrahydrothien-3-yl, Pyrid-3-yl, 1,3-Dioxepan-5-yl, Tetrahydro-pyrano-pyranyl gemäß Formel IIa, Tetrahydrothiino-thiinyl gemäß Formel IIa, Hexahydropyranopyranyl gemäß Formel II und Hexahydrothiinothiinyl gemäß Formel II, bedeutet.

4. Cyclohexenonderivate der Formel I gemäß Anspruch 1, in denen $R^3$ Thien-2-yl oder Thien-3-yl bedeutet, das gegebenenfalls jeweils durch ein oder zwei Chloratome substituiert ist.

5. Verfahren zur Herstellung von Cyclohexenonderivaten der Formel I gemäß Anspruch 1, dadurch gekennzeichnet, daß man eine Tricarbonylverbindung der Formel III

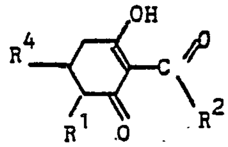

(III)

in der $R^1$, $R^2$ und $R^4$ die in Anspruch 1 genannte Bedeutung besitzen,

a) mit einer Ammoniumverbindung der Formel $R^3—CH_2O—NH_3Y$, in der $R^3$ die in Anspruch 1 genannte Bedeutung hat und Y ein Anion bedeutet, in einem inerten Verdünnungsmittel gegebenenfalls in Gegenwart von Wasser bei einer Temperatur zwischen 0 und 80 °C in Gegenwart einer Base oder

b) mit einem gegebenenfalls in wäßriger Lösung vorliegenden Hydroxylamin der Formel $R^3—CH_2O—NH_2$, in der $R^3$ die in Anspruch 1 genannte Bedeutung hat, in einem inerten Lösungsmittel umsetzt.

6. Herbizid, enthaltend ein Cyclohexenonderivat der Formel I gemäß Anspruch 1.

7. Herbizid, enthaltend inerte Zusatzstoffe und ein Cyclohexenonderivat der Formel I gemäß Anspruch 1.

8. Verfahren zur Bekämpfung unerwünschten Pflanzenwachstums, dadurch gekennzeichnet, daß man die unerwünschten Pflanzen und/oder die von unerwünschten Pflanzenwachstum freizuhaltende Fläche mit einer herbizid wirksamen Menge eines Cyclohexenonderivates der Formel I gemäß Anspruch 1 oder Salzen dieser Verbindung behandelt.

## Claims

1. A cyclohexenone derivative of the formula I

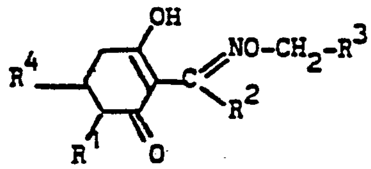

(I)

where

$R^1$ is hydrogen or alkoxycarbonyl of 2 to 5 carbon atoms,

$R^2$ is alkyl of 1 to 4 carbon atoms,

$R^3$ is a 5-membered heterocyclic structure which contains 1 to 3 heteroatoms from the group consisting of N, O and S and may contain 1 or 2 double bonds and 1 or 2 substituents from the group consisting of $C_1$-$C_4$ alkyl, $C_1$-$C_4$-alkoxy, halogen, trifluoromethyl, $C_1$-$C_4$-alkoxymethyl, $C_1$-$C_4$-alkylthiomethyl, vinyl and phenyl, and

$R^4$ is a 5-membered to 7-membered heterocyclic structure which contains one heteroatom or ring member, or two identical or different heteroatoms or ring members, from the group consisting of N, O, S, SO and $SO_2$, may contain 1, 2 or 3 double bonds and is unsubstituted or substitued by not more than 2 alkyl or alkoxy groups, each of 1 to 4 carbon atoms, or is a radical of the formula II

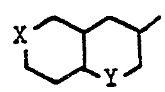

(II)

or of the formula IIa

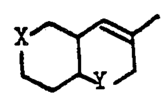

(IIa)

where X and Y are each N, O, S, SO or $SO_2$, or a salt thereof.

2. A cyclohexenone derivative of the formula I as claimed in claim 1, where $R^3$ is a substituent selected from the group consisting of furan-2-yl, tetrahydrofuran-2-yl, 1,3-dioxolan-2-yl, 2-phenyl-1,3-dioxolan-2-yl, 2,2-dimethyl-1,3-dioxolan-4-yl, 4,5-dimethyl-1,3-dioxolan-2-yl, 2-vinyl-1,3-dioxolan-4-yl, 3-phenylisoxazol-5-yl, 3-methylisoxazol-5-yl, 3-methoxymethylisoxazol-5-yl, 5-chlorothien-2-yl, 2-chlorothien-3-yl, 3,5-dichlorothien-2-yl, 2,5-dichlorothien-3-yl, 2,3-dichlorothien-4-yl, 2-chlorothien-4-yl, 2-isopropyl-1,3,4-oxadiazol-5-yl, 2-methyl-1,3,4-thiadiazol-5-yl, 5-chloro-1,2,3-thiadiazol-4-yl, thien-2-yl and thien-3-yl.

3. A cyclohexenone derivative of the formula I as claimed in claim 1, where $R^4$ is a substituent selected from the group consisting of tetrahydropyran-3-yl, tetrahydropyran-4-yl, tetrahydrothiopyran-3-yl, tetrahydrothiopyran-4-yl, tetrahydrofuran-3-yl, tetrahydrothien-3-yl, pyrid-3-yl, 1,3-dioxepan-5-yl, tetrahydropyranopyranyl according to formula IIa, tetrahydrothiinothiinyl according to formula IIa, hexahydropyranopyranyl according to formula II and hexahydrothiinothiinyl according to formula II.

4. A cyclohexenone derivative of the formula I as claimed in claim 1, where $R^3$ is thien-2-yl or thien-3-yl which is unsubstituted or substituted by one or two chlorine atoms.

5. A process for the preparation of a cyclohexenone derivative of the formula I as claimed in claim 1, wherein a tricarbonyl compound of the formula III

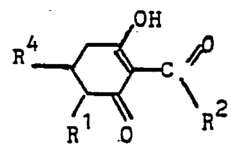

(III)

where $R^1$, $R^2$ and $R^4$ each have the meanings indicated in claim 1, is reacted

a) with an ammonium compound of the formula $R^3—CH_2O—NH_3Y$, where $R^3$ has the meanings indicated in claim 1 and Y is an anion, in an inert diluent, in the presence or absence of water, at from 0 to 80 °C and in the presence of a base, or

b) with a hydroxylamine — if desired, in aqueous solution — of the formula $R^3—CH_2O—NH_2$, where $R^3$ has the meanings indicated in claim 1, in an inert solvent.

6. A herbicide containing a cyclohexenone derivative of the formula I as claimed in claim 1.

7. A herbicide containing inert additives and a cyclohexenone derivative of the formula I as claimed in claim 1.

8. A method for controlling undesirable plant growth, wherein the undesirable plants or the area to be kept free from undesirable plant growth is treated with a herbicidally effective amount of a cyclohexenone derivative of the formula I as claimed in claim 1, or a salt thereof.

**Revendications**

1. Dérivés, et sels de ces dérivés, de cyclohéxénone de formule I

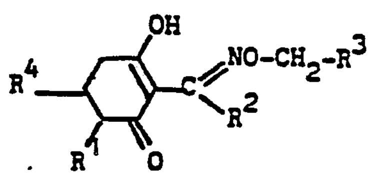

(I)

dans laquelle

$R^1$ représente hydrogène ou alcoxycarbonyle à 2 à 5 atomes C

$R^2$ alkyle à 1 à 4 atomes de carbone

$R^3$ un hétérocycle à 5 chaînons, qui contient 1 à 3 hétéroatomes du groupe N, O, S, éventuellement jusqu'à 2 doubles liaisons et éventuellement jusqu'à 2 substituants du groupe alkyle en $C_1$-$C_4$, alcoxy en $C_1$-$C_4$, halogène, trifluorométhyle, alcoxyméthyle en $C_1$-$C_4$, alkylthiométhyle en $C_1$-$C_4$, vinyle ou phényle,

$R^4$ un hétérocycle à 5 à 7 chaînons, qui contient 1 ou 2 hétéroatomes identiques ou différents ou des chaînons de noyau du groupe N, O, S, SO et $SO_2$, et éventuellement jusqu'à 3 doubles liaisons, et qui éventuellement est substitué par au maximum 2 groupes alkyle ou alcoxy ayant chacun 1 à 4 atomes de carbone, ou un reste de formule II

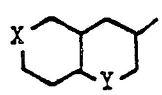

(II)

ou de formule IIa

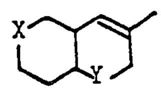

(IIa)

dans lesquelles X et Y sont mis chacun pour N, O, S, SO ou $SO_2$.

2. Dérivés de cyclohéxénone de formule I selon la revendication 1, dans lesquels $R^3$ représente un substituant, choisi dans le groupe comprenant furan-2-yle, tétrahydrofuran-2-yle, 1,3-dioxolan-2-yle, 2-phényl-1,3-dioxolan-2-yle, 2,2-diméthyl-1,3-dioxolan-4-yle, 4,5-diméthyl-1,3-dioxolan-2-yle, 2-vinyl-1,3-dioxolan-4-yle, 3-phényl-isoxazol-5-yle, 3-méthyl-isoxazol-5-yle, 3-méthoxyméthyl-isoxazol-5-yle, 5-chlorothien-2-yle, 2-chlorothien-3-yle, 3,5-dichlorothien-2-yle, 2,5-dichlorothien-3-yle, 2,3-dichlorothien-4-yle, 2-chlorothien-4-yle, 2-isopropyl-1,3,4-oxadiazol-5-yle, 2-méthyl-1,3,4-thiadiazol-5-yle, 5-chloro-1,2,3-thiadiazol-4-yle, thien-2-yle et thien-3-yle.

3. Dérivés de cyclohéxénone de formule I selon la revendication 1, dans lesquels $R^4$ représente un substituant choisi dans le groupe comprenant tétrahydropyran-3-yle, tétrahydropyran-4-yle, tétrahydrothiopyran-3-yle, tétrahydrothiopyran-4-yle, tétrahydrofuran-3-yle, tétrahydrothien-3-yle, pyrid-3-yle, 1,3-dioxepan-5-yle, tétrahydropyrano-pyranyle selon la formule IIa, tétrahydrothiinothiinyle selon la

formule IIa, héxahydropyranopyranyle selon la formule II et hexahydrothiinothiinyle selon la formule II.

4. Dérivés de cyclohéxénone de formule I selon la revendication 1, dans lesquels $R^3$ représente thien-2-yle ou thien-3-yle qui est éventuellement substituée respectivement par un ou deux atomes de chlore.

5. Procédé de préparation de dérivés de cyclohéxénone de formule I selon la revendication 1, caractérisé par le fait que l'on fait réagir un composé tricarbonyle de formule III

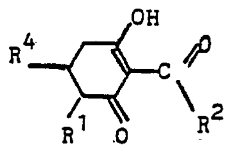

(III)

dans laquelle $R^1$, $R^2$ et $R^4$ ont la signification donnée dans la revendication 1,

a) avec un composé d'ammoniac de formule $R^3—CH_2O—NH_3Y$, dans laquelle $R^3$ a la signification donnée dans la revendication 1 et Y représente un anion, dans un diluant inerte, éventuellement en présence d'eau, à une température comprise entre 0 et 80 °C, en présence d'une base, ou

b) avec une hydroxylamine de formule $R^3—CH_2O—NH_2$ se trouvant éventuellement en solution aqueuse, où $R^3$ a la signification indiquée dans la revendication 1, dans un solvant inerte.

6. Herbicide, contenant un dérivé de cyclohéxénone de formule I selon la revendication 1.

7. Herbicide, contenant des additifs inertes et un dérivé de cyclohéxénone de formule I selon la revendication 1.

8. Procédé de lutte contre la croissance des plantes indésirables, caractérisé par le fait que l'on traite les plantes indésirables et/ou les surfaces à maintenir libres d'une croissance indésirable des plantes avec une quantité efficace, du point de vue herbicide, d'un dérivé de cyclohéxénone de formule I selon la revendication 1, ou de sels de ce composé.